(19) Europäisches Patentamt
European Patent Office
Office européen des brevets

(11) Numéro de publication: **0 256 906 B1**

(12) # FASCICULE DE BREVET EUROPEEN

(45) Date de publication de fascicule du brevet: **04.12.91**

(51) Int. Cl.5: **A61L 27/00**, C08B 16/00

(21) Numéro de dépôt: **87401640.5**

(22) Date de dépôt: **10.07.87**

(54) **Matériau hydrophyle biocompatible, et son procédé de fabrication.**

(30) Priorité: **16.07.86 FR 8610331**

(43) Date de publication de la demande:
**24.02.88 Bulletin 88/08**

(45) Mention de la délivrance du brevet:
**04.12.91 Bulletin 91/49**

(84) Etats contractants désignés:
**AT BE CH DE ES FR GB GR IT LI LU NL SE**

(56) Documents cités:
**WO-A-86/01113**
**FR-A- 1 310 496**
**FR-A- 2 147 139**
**GB-A- 407 811**
**US-A- 3 108 357**

**GAZETTE VAN ANTWERPEN, 8 novembre 1977, page 7; "Houtenbeen": "Le tissu osseux repousse dans les pores de diverses sortes de bois notamment le bois de frêne ou de bouleau"**

(73) Titulaire: **LA CELLULOSE DU PIN**
**353 rue du Président Wilson**
**F-33100 Bordeaux(FR)**

Titulaire: **UNIVERSITE DE BORDEAUX II**
**146, rue Léo Saignat**
**F-33076 BordeauxCédex(FR)**

(72) Inventeur: **Pommier, Jean-Claude**
**35 Allée de Gascogne**
**F-33170 Gradignan(FR)**
Inventeur: **Poustis, Joel**
**3 Allée de Saintonge Cap de Bos**
**F-33600 Pessac(FR)**
Inventeur: **Bacquey, Charles**
**33 Rue du Médoc Le Haillan**
**F-33160 St Médard en Jalles(FR)**
Inventeur: **Chauveaux, Dominique**
**13 Rue Marceaux**
**F-33000 Bordeaux(FR)**

(74) Mandataire: **Muller, René**
**SAINT-GOBAIN RECHERCHE 39, quai Lucien Lefranc**
**F-93304 Aubervilliers(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention concerne un matériau hydrophile biocompatible, et son procédé de fabrication.

En face d'une lacune osseuse d'origine pathologique ou traumatique, dont la pérennité peut avoir des conséquences biomécaniques ou même esthétiques, le médecin se doit d'intervenir pour essayer de promouvoir la reconstitution de l'os manquant ou de suppléer ce manque par la pratique d'une greffe osseuse ou par l'apposition d'un biomatériau convenablement choisi.

La pratique de l'autogreffe est une solution, mais les sources utilisables (côtes, crêtes iliaques, os spongieux épyphysaire) sont limitées quant aux volumes disponibles et leur exploitation n'est pas toujours compatible avec l'état de santé des patients. A défaut d'autogreffe, le chirurgien peut pratiquer une homogreffe s'il dispose à proximité d'une banque bien gérée et bien approvisionnée ; en tout état de cause, la disponibilité quantitative reste limitée.

Ainsi la disponibilité de substituts de masse osseuse d'origine allogène apparait clairement comme la seule solution permettant de répondre avec souplesse et efficacité aux besoins. Les matériaux proposés pour la confection de tels substituts sont généralement composites et font intervenir dans leur composition une phase minérale d'origine naturelle comme le corail ou plus souvent d'origine synthétique tels l'hydroxyapatite, le phosphate tricalcique ou le phosphate octacalcique et une phase organique servant de liant et propice à la colonisation cellulaire à partir des tissus d'accueil adjacents.

Les nombreux travaux menés sur le sujet mettent pour la plupart en évidence l'intérêt de tels mélanges composites pour le comblement des pertes de substance osseuse, mais les procédés de préparation et de mise en oeuvre proposés ne sont pas entièrement satisfaisants. En outre, le coût des matières premières interdit leur emploi pour le comblement de cavités volumiquement importantes, rencontrées en traumatologie ou en carcinologie.

Un autre domaine d'utilisation d'un matériau biocompatible est le domaine des prothèses articulaires.

Les prothèses actuelles réalisées en un matériau métallique ou de synthèse peuvent être scellées dans l'os par un liant tel un ciment acrylique. Un des inconvénients de ce tue de scellement est l'atrophie des cellules v'vantes voisines due à la température de polymérisation du ciment.

Selon une variante, les prothèses peuvent aussi être scellées par ancrage biologique. Dans ce cas, on observe une repousse osseuse qui au cas où la prothèse doit être retirée, notamment en cas de rupture de celle-ci, complique son retrait.

Le document FR-A-2 147 139 décrit un procédé de fabrication de cellulose régénérée et activée par incorporation d'un composant actif dans le but d'obtenir une matière échangeuse d'ions. Ce document ne décrit pas un procédé pour obtenir un matériau à base de cellulose sous forme d'un bloc qui soit biocompatible.

Le document GB-A-407 811 décrit un traitement de désulfuration de tubes à parois minces en cellulose régénérée, utilisés en particulier en tant que peaux de saucisses.

Les documents FR-A-1 310 496, US-A-3 108 357, WO-A-8 601 113 décrivent des matériaux à base de cellulose qui sont biodégradables.

L'invention propose un matériau pour un scellement réversible qui obvie aux inconvénients cités. Le matériau pour le scellement selon l'invention est un matériau biocompatible présentant un potentiel d'expansion volumique susceptible de créer des efforts normaux aux surfaces en regard et qui s'opposent aux mouvements relatifs de la pièce prothétique et de l'os prothésé. Le matériau selon l'invention placé entre l'élément de prothèse et la partie réceptrice de l'os fournit un scellement à "joint d'effort modulable". Lorsqu'on libère le potentiel évoqué ci-dessus, l'expansion résultante s'accompagne d'une dégradation des propriétés mécaniques du matériau, ce qui facilite le retrait de la prothèse.

Un des objets de l'invention est un matériau biocompatible hydrophile qui présente les propriétés pour pouvoir être utilisé notamment pour la confection de substitut de masse osseuse ou pour la confection de gaines d'ajustage pour des prothèses articulaires.

Un matériau conforme à l'invention est un matériau à base de cellulose purifiée, matériau duquel on a retiré par séchage la totalité ou la majeure partie de l'eau, et qui présente un potentiel de reprise d'eau modulable jusqu'à une teneur en eau de 60 %.

Ce matériau est obtenu en particulier par la suite des opérations suivantes :

- on prépare de l'alcali-cellulose par trempage de la pâte de cellulose notamment de la pâte obtenue par le procédé bisulfite acide, dans une solution de soude suivi d'un essorage et d'un pressage pour atteindre un poids en cellulose sèche compris entre 30 % et 40 %,
- on réalise ensuite la sulfuration de l'alcali-cellulose par du sulfure de carbone, de préférence en excès par rapport aux quantités stoéchiométriques, pour obtenir du xanthate de sodium,
- on dissous le xanthate de sodium dans une solution de soude diluée pour obtenir de la viscose, c'est-à-dire une solution contenant de la cellulose et de la soude à des teneurs bien déterminées, de 7 à 12 % en poids de cellulose et de 4 à 8 % en poids de soude, caractérisé en ce qu'on soumet la solution visqueuse obtenue à un traitement thermique afin de provoquer la coagulation de la viscose et à un traitement d'élimination du soufre, ces deux traitements s'effectuant par phases de façon alternée, pour obtenir un matériau amorphe et présentant un caractère caoutchouteux, et une teneur en soufre inférieure á 0,1%,

- et on sèche le matériau obtenu.

Selon une forme de réalisation du procédé, on provoque d'abord une coagulation partielle de la solution visqueuse par un traitement thermique, par exemple en portant ladite solution à environ 100°C pendant environ 1-2 heures, puis on élimine le soufre par lavage, avec de l'eau et avec un produit réactif vis-à-vis du soufre tel le sulfite de sodium, le carbonate de sodium. Afin d'augmenter l'efficacité du lavage, de préférence, on peut prévoir un chauffage ou traitement du bloc coagulé dans le bain de lavage, par des micro-ondes.

Dans une variante du procédé, on réalise un traitement d'élimination partielle du soufre avant d'effectuer la coagulation par un traitement thermique.

On s'est aperçu qu'une coagulation trop importante de la solution de viscose rendait l'élimination ultérieure du soufre difficile. Et qu'à l'inverse, une élimination trop importante du soufre avant la coagulation rendait celle-ci également difficile. Ainsi, on préfère effectuer une élimination partielle du soufre en traitant la solution de viscose avec un agent réactif avec le soufre tel le sulfite de sodium et le carbonate de sodium par exemple à raison d'environ 10 g de sulfite de sodium et 2 g de carbonate de sodium pour 100 $cm^3$ de solution de viscose. Puis on démarre la coagulation. On lave avec de l'eau et on recommence cette suite d'opérations.

Ce procédé permet d'atteindre des taux de soufre bien inférieurs à 0,1 % en poids du poids du matériau.

Selon une caractéristique du procédé, le séchage est réalisé par un pressage à chaud du matériau obtenu après coagulation de la cellulose. A cet effet, le matériau peut être placé dans une étuve à une température de 110°C et sous une pression de 1 à 10 bars pendant 24 heures.

Le matériau obtenu peut être facilement usiné pour l'obtention de toutes formes de pièces. En effet, ce matériau présente des propriétés mécaniques proches de celles du bois et il peut être usiné de façon semblable et même mieux, car à la différence du bois, ce matériau est isotrope.

Ce matériau fortement hydrophile est capable d'une reprise en eau équivalente à 60 % de son poids pour 90 % d'augmentation de volume et perd dans ce cas toute tenue mécanique. Mais lorsque le volume d'expansion offert est nul ou limité, l'absorption d'eau est nécessairement modérée, allant jusqu'à 20 % en poids et la perte de propriétés mécaniques reste acceptable pour les applications envisagées.

Sous un des aspects avantageux de l'invention, on incorpore au matériau, au cours de sa préparation, des fibres de renforcement notamment des fibres papetières, de préférence avant l'opération de coagulation de la cellulose. Le taux de fibres incorporées que l'on retrouve dans le matériau obtenu peut varier de 0 à 50 % en poids par rapport au poids total du matériau composite. L'incorporation des fibres sus-mentionnées améliore certaines caractéristiques du matériau, en particulier la résistance à la fatigue.

Une des applications particulièrement avantageuse de ce matériau, qui en exploite les propriétés évoquées précédemment, conformément à l'invention, est la confection d'une gaine d'ajustage pour des prothèses articulaires, en particulier pour les queues de prothèse de hanche permettant de réaliser leur ancrage réversible dans le canal médullaire du fémur.

Le matériau est suffisamment biocompatible pour ne pas entrainer de lésions ni de nécroses sur les tissus vivants avec lesquels il doit être interfacé.

Une autre qualité du matériau est qu'il permet un ajustement résistant aux mouvements de rotation d'une part et de translation axiale d'autre part de la queue de prothèse de hanche dans le canal médullaire du fémur. Grâce à son potentiel de reprise d'eau, le matériau reprend de l'eau fournie en quantité déterminée au cours du scellement. De par l'expansion volumique qui lui est liée, il va s'établir des forces de pressions qui vont s'opposer aux mouvements relatifs de la prothèse et de la pièce osseuse réceptrice.

Grâce à ses propriétés, notamment à son potentiel de reprise d'eau, le matériau autorise aussi une mise en place réversible des prothèses et supprime les inconvénients des prothèses scellables par ciment ou des prothèses auto-scellables dont le retrait est particulièrement délicat, notamment en cas de rupture de la prothèse. Ainsi pour retirer une prothèse conforme à l'invention, il suffit de fournir un supplément d'eau artificiellement à la gaine qui va ainsi perdre ses propriétés de résistance mécanique.

Une variante pour la préparation du matériau hydrophile permet d'obtenir directement des pièces de formes multiples. Cette variante consiste à mouler le matériau obtenu après coagulation de la cellulose tout en extrayant l'eau, en tenant compte des retraits correspondant à l'élimination de la majeure partie de l'eau.

Le matériau hydrophile selon l'invention peut également être utilisé pour la confection de substitut de masse osseuse. Selon une caractéristique de l'invention, on peut modifier chimiquement la surface limitante du matériau, de sorte que les cellules du tissu d'accueil puissent le coloniser de façon contrôlée. On peut aussi moduler ou améliorer la cytocompatibilité du matériau par une réticulation et/ou un greffage radiochimique.

D'autres avantages et caractéristiques de l'invention apparaitront dans les exemples décrits ci-après.

EXEMPLE 1

Cet exemple décrit une préparation du matériau poreux pouvant être utilisé notamment pour la confection de gaines d'ajustage de queue de prothèse.

On traite 650 g de pate de cellulose par une solution de soude 6 M selon la réaction.

$$(6_6H_{10}O_5)_n + nNaOH \rightarrow (C_6H_9O_4\ \text{-ONa})_n + nH_2O$$

On élimine ainsi les impuretés telles les hémi-celluloses résiduaires, les résines et on forme l'alcali-cellulose. On contrôle le rapport cellulose/NaOH, en pressant la suspension jusqu'à atteindre un poids en cellulose sèche de 35 % et en éliminant une partie de la soude en excès et des hémi-celluloses dissoutes.

On prépare ensuite le xanthate par sulfuration de l'alcali-cellulose par le sulfure de carbone $CS_2$ dans un réacteur en utilisant du $CS_2$ à raison de 31 % en poids du poids de la cellulose, selon la réaction

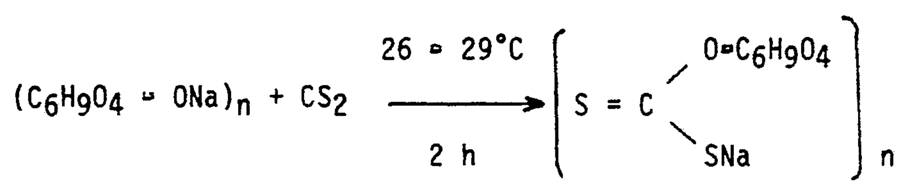


On dissous ensuite le xanthate dans une solution de soude diluée. En fin d'opération, la solution contient une teneur en cellulose de 9 % et de soude de 5 %. La viscose obtenue est désaérée. On chauffe la solution à 100 °C pendant 2 heures ce qui provoque une coagulation. On lave ensuite plusieurs fois avec de l'eau, du sulfite et du carbonate de soude. On obtient un matériau qui se présente sous une forme compacte, amorphe et relativement caoutchouteuse. Ce matériau est ensuite pressé durant 24 heures à une température de 110 °C sous une pression de 2 bars, ce qui élimine l'eau qu'il contient.

On obtient un matériau qui a l'aspect d'un bois naturel.

Ce matériau hydrophile obtenu peut être utilisé selon l'invention dans diverses applications. Ses propriétés mécaniques telle que la rigidité en flexion, compression et tension sont comparables à celles d'un bois naturel. Ce matériau isotrope peut être usiné facilement.

A partir du matériau hydrophile obtenu, on usine une gaine d'ajustage pour une queue métallique de prothèse de hanche afin d'appliquer le concept d'un scellement à joint d'effort modulable.

On réalise un assemblage modèle constitué par un tronçon de diaphyse fémorale d'origine humaine dont le canal est occupé par la pièce métallique constituant la queue de prothèse, entourée par la gaine usinée du matériau poreux. Un des avantages du matériau utilisé est qu'on peut ajuster aisément le rapport d'occupation du canal par la gaine et optimiser la résistance de l'assemblage.

L'ensemble est immergé dans du sérum physiologique à 37 °C. L'empêchement de l'expansion volumique se traduit par l'établissement de pressions normales à l'interface limitant l'expansion.

Sur la figure 1, on a représenté très schématiquement l'articulation de la hanche 1 avec 1a queue de prothèse 2 entourée par la gaine usinée 3 du matériau hydrophile selon l'invention, en place dans le canal médullaire du fémur 4.

L'assemblage résiste bien aux efforts statique de cisaillements longitudinaux et rotationnels. La gaine contribue également à l'amortissement des nombreuses sollicitations dynamiques auxquelles est confrontée la prothèse articulaire.

Le module d'élasticité mesuré du matériau hydrophile est compris entre 7 et 12 gigapascals alors que celui d'un ciment traditionnel en polyméthacrylate de méthyle est compris entre 5,1 et 5,7 gigapascals. Le matériau selon l'invention est moins rigide et il encaisse mieux les chocs.

EXEMPLE 2

On opère de la même façon que dans l'exemple 1, sauf qu'on incorpore à la solution de viscose avant l'opération de coagulation, des fibres papetières de renforcement, à raison de 30 % en poids de la viscose.

Le produit obtenu peut être usiné aussi facilement que le produit hydrophile selon l'exemple 1.

La résistance à la fatigue est améliorée.

EXEMPLE 3

On opère de la même façon que dans l'exemple 1, sauf qu'on soumet la viscose à un traitement d'élimination partielle du soufre, avant l'opération de coagulation. A cette fin, on ajoute à la solution de viscose du sulfite de sodium et du carbonate de sodium à raison de 10 g de sulfite de sodium et 2 g de carbonate de sodium pour 100 $cm^3$ de solution de viscose. On laisse sous agitation à la température ambiante. On démarre ensuite la coagulation en chauffant la solution à 100° C environ pendant 1 heure environ. Le bloc obtenu est lavé plusieurs fois avec de l'eau chaude et de l'eau froide.

Après séchage, on obtient un matériau qui a l'aspect du bois naturel et dont la teneur en soufre est de 0,09 %.

**Revendications**

1. Matériau hydrophile biocompatible formé essentiellement de cellulose purifiée, utilisable dans le corps humain, caractérisé en ce que le matériau se présente en forme de bloc, matériau duquel on a retiré par séchage la totalité ou la majeure partie de l'eau, qu'il présente un potentiel de reprise d'eau modulable jusqu'à une teneur en eau de 60 %, la reprise d'eau étant liée à une expansion volumique, et une teneur en soufre inférieure à 0,1 % en poids.

2. Matériau selon la revendication 1, caractérisé en ce que le matériau contient des fibres de renforcement.

3. Matériau selon la revendication 2, caractérisé en ce que les fibres de renforcement du matériau sont des fibres papetières.

4. Matériau selon une des revendications 1 à 3, caractérisé en ce que la surface limitante du matériau est modifiée de sorte que les cellules du tissu d'accueil puissent le coloniser de façon contrôlée.

5. Matériau selon une des revendications 1 à 3, caractérisé en ce qu'il est utilisé pour la confection de gaines d'ajustage pour des prothèses articulaires.

6. Matériau selon une des revendications 1 à 4, caractérisé en ce qu'il est utilisé pour la confection de substitut de masse osseuse.

7. Procédé pour la fabrication d'un matériau hydrophile biocompatible en forme de bloc comprenant les opérations suivantes :
   - on prépare de l'alcali-cellulose par trempage de la pâte de cellulose notamment de la pâte obtenue par le procédé bisulfite acide, dans une solution de soude suivi d'un essorage et d'un pressage pour atteindre un poids en cellulose sèche compris entre 30 % et 40 %,
   - on réalise ensuite la sulfuration de l'alcali-cellulose par du sulfure de carbone, de préférence en excès par rapport aux quantités stoéchiométriques, pour obtenir du xanthate de sodium,
   - on dissous le xanthate de sodium dans une solution de soude diluée pour obtenir de la viscose, c'est-à-dire une solution contenant de la cellulose et de la soude à des teneurs bien déterminées, de 7 à 12 % en poids de cellulose et de 4 à 8 % en poids de soude, caractérisé en ce qu'on soumet la solution visqueuse obtenue à un traitement thermique afin de provoquer la coagulation de la viscose et à un traitement d'élimination du soufre, ces deux traitements s'effectuant par phases de façon alternée, pour obtenir un matériau amorphe présentant un caractère caoutchouteux, et une teneur en soufre inférieure à 0,1 %,
   - et on sèche le matériau obtenu pour lui retirer la totalité ou la majeure partie de l'eau et pour lui procurer un potentiel de reprise en eau.

8. Procédé selon la revendication 7, caractérisé en ce qu'on provoque d'abord une coagulation partielle de la cellulose par un traitement thermique, avant de procéder à une élimination du soufre par lavage

avec de l'eau et un produit réactif avec le soufre, notamment le sulfite de sodium, le carbonate de sodium.

9. Procédé selon la revendication 8, caractérisé en ce que l'élimination du soufre par lavage est effectuée en présence d'un chauffage par micro-ondes.

10. Procédé selon la revendication 7, caractérisé en ce qu'on élimine partiellement le soufre de la solution de viscose par traitement avec un produit réactif avec le soufre notamment le sulfite de sodium, le carbonate de sodium, avant de provoquer la coagulation.

11. Procédé selon une des revendications 7 à 10, caractérisé en ce que l'eau est éliminée par pressage à chaud.

12. Procédé selon une des revendications 7 à 11, caractérisé en ce que l'eau est éliminée dans un moule de façon à obtenir directement un produit ayant la forme désirée.

13. Procédé selon une des revendications 7 à 12, caractérisé en ce qu'on incorpore des fibres de renforcement dans la solution de viscose.

## Claims

1. Biocompatible hydrophilic material, composed essentially of purified cellulose, capable of being used in the human body, characterized in that the material is present in block form, from which material the totality or the greater part of the water has been removed by drying, that it has a water reabsorption potential which can be modulated up to a water content of 60%, the water absorption being linked to a volumetric expansion, and a sulphur content less than 0.1% by weight.

2. Material according to Claim 1, characterized in that the material contains reinforcing fibres.

3. Material according to Claim 2, characterized in that the reinforcing fibres of the material are paper fibres.

4. Material according to one of Claims 1 to 3, characterized in that the limiting surface of the material is modified in such a way that the cells of the receiving tissue can colonize it in controlled manner.

5. Material according to one of Claims 1 to 3, characterized in that it is used for making fitting sheaths for articular prostheses.

6. Material according to one of Claims 1 to 4, characterized in that it is used for making bony material substitute.

7. Process for the production of a biocompatible hydrophilic material in block form, comprising the following operations:
   - alkali-cellulose is prepared by soaking cellulose pulp, notably pulp obtained by the acid bisulphite process, in a soda solution followed by draining and pressing to achieve a dry cellulose weight from 30% to 40%,
   - sulphurization of the alkali-cellulose is then carried out by carbon disulphide, preferably in excess of the stoichiometric quantities, to obtain sodium xanthate,
   - the sodium xanthate is dissolved in a dilute soda solution to obtain viscose, that is to say a solution containing cellulose and soda in well determined quantities, from 7 to 12% by weight cellulose and from 4 to 8% by weight soda, characterized in that the viscose solution obtained is subjected to a heat treatment in order to cause coagulation of the viscose and to a treatment for removal of the sulphur, these two treatments being carried out by alternating phases, in order to obtain an amorphous material having a rubbery character, and a sulphur content less than 0.1%,
   - and the material obtained is dried to remove from it the totality or the greater part of the water and to give it a water reabsorption potential.

8. Process according to Claim 7, characterized in that a partial coagulation of the cellulose is first brought

about by a heat treatment, before proceeding to a removal of the sulphur by washing with water and a product that reacts with sulphur, notably sodium sulphite, sodium carbonate.

**9.** Process according to Claim 8, characterized in that the removal of the sulphur by washing is carried out in the presence of microwave heating.

**10.** Process according to Claim 7, characterized in that the sulphur is partially removed from the viscose solution by treatment with a product that reacts with sulphur, notably sodium sulphite, sodium carbonate, before causing the coagulation.

**11.** Process according to one of Claims 7 to 10, characterized in that the water is removed by hot pressing.

**12.** Process according to one of Claims 7 to 11, characterized in that the water is removed in a mould, in order to obtain directly a product having the desired shape.

**13.** Process according to one of Claims 7 to 12, characterized in that reinforcing fibres are incorporated into the viscose solution.

**Patentansprüche**

**1.** Hydrophiler, biokompatibler, im wesentlichen aus gereinigter Zellulose gebildeter, im menschlichen Körper verwendbarer Werkstoff, **dadurch gekennzeichnet, daß** der Werkstoff, dem durch Trocknen das Wasser ganz oder größtenteils entzogen wurde, in Blockform vorliegt, ein bis zu einem Wassergehalt von 60% variierbares Wasseraufnahmepotential aufweist, wobei die Wasseraufnahme mit einer volumenmäßigen Expansion verbunden ist, **und daß** der Werkstoff einen Schwefelgehalt von weniger als 0,1 Gewichts-% aufweist.

**2.** Werkstoff nach Anspruch 1, **dadurch gekennzeichnet, daß** der Werkstoff Verstärkungsfasern enthält.

**3.** Werkstoff nach Anspruch 2, **dadurch gekennzeichnet, daß** die Verstärkungsfasern des Werkstoffs papierene Fasern sind.

**4.** Werkstoff nach einem der Ansprüche 1 bis 3, **dadurch gekennzeichnet, daß** die Begrenzungsfläche des Werkstoffes derart modifiziert ist, daß die Zellen des aufnehmenden Gewebes diesen auf kontrollierte Weise kolonisieren können.

**5.** Werkstoff nach einem der Ansprüche 1 bis 3, **dadurch gekennzeichnet, daß** er für die Anfertigung von Justierungshüllen für Gelenkprothesen verwendet wird.

**6.** Werkstoff nach einem der Ansprüche 1 bis 4, **dadurch gekennzeichnet, daß** er für die Anfertigung von Ersatz für Knochenmasse verwendet wird.

**7.** Verfahren zur Herstellung eines hydrophilen, biokompatiblen blockförmigen Werkstoffes, bestehend aus folgenden Arbeitsgängen:

- es wird Alkali-Zellulose hergestellt durch Härten der Zellulose-Masse, insbesondere der durch das Bisulfitsäure-Verfahren erhaltenen Masse, in einer Sodalösung, danach wird die Masse getrocknet und gepreßt, um ein Gewicht der trockenen Zellulose zwischen 30% und 40% zu erhalten,
- anschließend wird die Schwefelung der Alkali-Zellulose durch Schwefelkohlenstoff, vorzugsweise im Überschuß bezügl. Den stöchiometrischen Mengen, durchgeführt, um Natriumxanthat zu erhalten,
- das Natriumxanthat wird in einer verdünnten Sodalösung aufgelöst, um Viskose zu erhalten, d.h. eine Lösung, die Zellulose und Soda in genau festgelegten Anteilen enthält, nämlich 7 bis 12 Gewichts-% an Zellulose und 4 bis 8 Gewichts-% an Soda, **dadurch gekennzeichnet, daß** die erhaltene viskose Lösung einer Wärmebehandlung zur Erzielung einer Koagulation der Viskose und einer Behandlung zur Entfernung des Schwefels unterzogen wird, wobei diese beiden Behandlungen in abwechselnden Phasen stattfinden, um einen amorphen Werkstoff mit kautschukartigem Charakter und einem Schwefelgehalt von weniger als 0,1% zu erhalten,
- der erhaltene Werkstoff wird getrocknet, um ihm das Wasser ganz oder größtenteils zu entziehen und um ihm ein Wasseraufnahmepotential zu verschaffen.

**8.** Verfahren nach Anspruch 7, **dadurch gekennzeichnet, daß** zuerst durch eine Wärmebehandlung eine partielle Koagulation der Zellulose hervorgerufen wird, bevor man zu einer Entfernung des Schwefels durch Spülung mit Wasser und einem mit dem Schwefel reagierenden Stoff, insbesondere Natriumsulfit, Natriumcarbonat, übergeht.

**9.** Verfahren nach Anspruch 8, **dadurch gekennzeichnet, daß** die Entfernung des Schwefels durch Spülung bei einer Mikrowellenbeheizung durchgeführt wird.

**10.** Verfahren nach Anspruch 7, **dadurch gekennzeichnet, daß** durch Behandlung mit einem mit dem Schwefel reagierenden Stoff, insbesondere Natriumsulfit, Natriumcarbonat, eine partielle Entfernung des Schwefels aus der Viskoselösung durchgeführt wird, bevor die Koagulation hervorgerufen wird.

**11.** Verfahren nach einem der Ansprüche 7 bis 10, **dadurch gekennzeichnet, daß** das Wasser durch Warmpressen entfernt wird.

**12.** Verfahren nach einem der Ansprüche 7 bis 11, **dadurch gekennzeichnet, daß** die Entfernung des Wassers in einer Form stattfindet, so daß man direkt ein Produkt in der gewünschten Gestalt erhält.

**13.** Verfahren nach einem der Ansprüche 7 bis 12, **dadurch gekennzeichnet, daß** der Viskoselösung Verstärkungsfasern beigemengt werden.

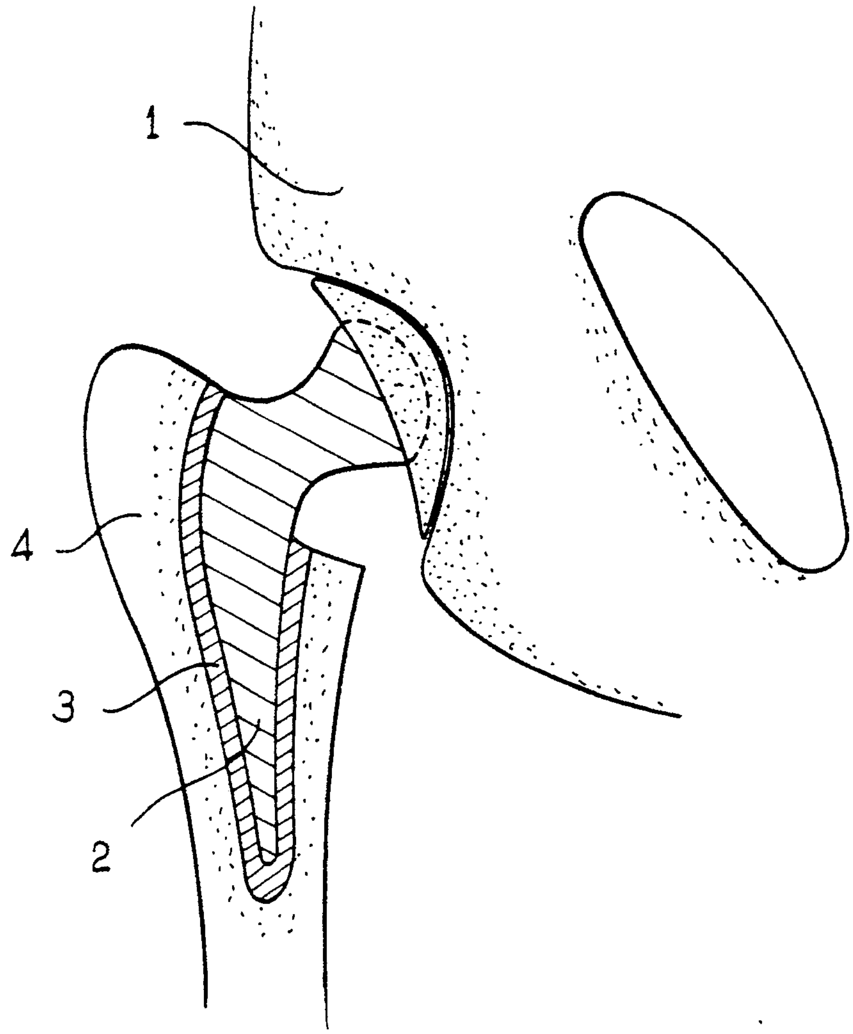
1
4
3
2